Europäisches Patentamt

(19) European Patent Office

Office européen des brevets

(11) Publication number: **0 089 135**
**A1**

(12) **EUROPEAN PATENT APPLICATION**

(21) Application number: 83301046.5

(22) Date of filing: 28.02.83

(51) Int. Cl.³: **A 61 M 1/03**

(30) Priority: 17.03.82 US 359015

(43) Date of publication of application:
21.09.83 Bulletin 83/38

(84) Designated Contracting States:
DE FR GB IT

(71) Applicant: THE CURATORS OF THE UNIVERSITY OF MISSOURI

Columbia Missouri(US)

(72) Inventor: Nolph, Karl D.
908 Hickory Hall
Columbia Missouri 65201(US)

(72) Inventor: Lang, Howard L.
16 Benwood Lane
Creve Coeur Missouri 63141(US)

(74) Representative: MacGregor, Gordon et al,
ERIC POTTER & CLARKSON 14 Oxford Street
Nottingham, NG1 5BP(GB)

(54) Peritoneal dialysis solution containing sorbents.

(57) A peritoneal dialysis solution which contains a sufficient amount of physiological salt to be osmotically compatible, characterised in that it also contains from 5 to 100 grams per liter of a particulate sorbent for metabolic breakdown products to be removed having a particle size of 0.5 to 2,000 microns.

EP 0 089 135 A1

Croydon Printing Company Ltd.

## PERITONEAL DIALYSIS
## SOLUTION CONTAINING SORBENTS

### Technical Field

Peritoneal dialysis is a technique of increasing popularity for the treatment of patients who are deficient in their kidney function for the removal of metabolic breakdown products from the bloodstream. An alternate technique for this treatment is hemodialysis, in which bood is removed from the patient, passed through a hemodialysis machine, then being returned to the patient. In peritoneal dialysis, the dialysis solution is conveyed to the peritoneal cavity, where it is allowed to dwell for a period of time during which diffusion exchange takes place between the dialysis solution and blood vessels surrounding the peritoneal cavity.

It has been stated that continuous ambulatory peritoneal dialysis (CAPD) offers a six-fold increase of weekly clearances of high molecular weight solutes, when compared with standard hemodialysis techniques. However, it is also said that clearances of low molecular weight solutes such as creatinine and urea are less than one- sixth that of hemodialysis.

One method of increasing the clearance of low molecular weight solutes in CAPD would be to increase the number of exchanges performed per day. Typically about four exchanges are performed on a dialy basis. However, this might increase not only the inconvenience to the patient, but the risk of peritonitis as well, which may be the most serious risk of the peritoneal dialysis technique.

The invention of this application proposes a technique for improvement in the absorption capacity of peritoneal dialysis solution, as a technique for increasing the clearance of particularly the lower molecular weight solutes in any peritoneal dialysis technique, but

preferably CAPD. Also ultrafiltration can be increased by this invention.

## Description of Prior Art

Chang U.S. Patent No. 3,522,346 discloses nonthrombogenic microcapsules which may contain urease, activated charcoal, or the like, surrounded by a coating of collodion or other polymeric macromolecular semipermeable membrane, the microcapsules being typically of the size range from 1 to 200 microns mean diameter.

Chang U.S. Patent No. 3,725,113 teaches blood compatible microencapsulated toxicants suitable for the detoxification of blood in an extracorporeal shunt, being made by coating a solid detoxicant with a semipermeable polymer membrane, and then coating the membrane with a noninterfering layer of a blood-compatible protein.

Activated charcoal has been previously used in some dialyzate regeneration systems as described in the Gordon et al. article entitled "Sorbents Regneration of Dialyzate", Kidney International, 10:S277-S283 (1976).

Also, encapsulated enzymes have been used to treat peritoneal fluid which is recirculated continuously through an extracorporeal system containing sorbent: T.M.S. Chang, Artificial Cells, published by Charles C. Thomas, Springfield, Illinois, pp. 114-115 (1972). In the same reference, at pp. 71-77 encapsulated urease and hemoglobin are reported to have been injected intraperitoneally.

## Description of the Invention

In accordance with this invention, a particulate sorbent material such as charcoal, and preferably microencapsulated charcoal, is added to peritoneal dialysis solution. While such items have been previously added to hemodialysis solution which does not enter a body cavity, the concept of this invention is to directly insert such particulate sorbent into the peritoneal dialysis solution, to allow the solution to dwell in the cavity for a period of time, and then to remove said solution and particulate sorbent from the peritoneal cavity.

Apart from containing desired sorbent in accordance with this invention, the peritoneal dialysis solution may be made in accordance with current practice for the formulation of peritoneal dialysis solution, containing appropriate quantities of physiological ionic salts and an osmotic agent, for example a sugar such as dextrose, but also optionally glycerol, polyglucose, or another osmotic agent for promoting ultrafiltration as may be desired as a partial or complete replacement for the sugar ingredient.

It also may be desired to add other nutrients and additives, for example amino acids or polypeptides to increase the osmolarity of the solution, and also to serve as a source of nutrition for the patient, to prevent the patient from being depleted of amino acids by the peritoneal dialysis process.

Conventional peritoneal dialysis solution thus may be used containing the preferably microencapsulated sorbents used in this invention. For example, a Dianeal® peritoneal dialysis solution sold by Travenol Laboratories, Inc. may have 40 grams per liter of collodion-encapsulated charcoal particles of 5 to 20 microns size dispersed therein for use in peritoneal dialysis.

It has been found that encapsulated carbon particles, and particularly activated charcoal, will bond creatinine, so that its presence in dialysis solution can greatly increase the clearance of creatinine and similar metabolic breakdown products.

The sorbent particles such as charcoal used herein may typically range from 0.5 to 2,000 microns in diameter, the smaller charcoal particles having larger surface area and thus tending to be more absorption efficient.  Thus the preferable range is about 1 to 20 microns, or up to 100 microns.  Encapsulation of the charcoal and other sorbent granules may be made in accordance with the method described in the article by Chang entitled "Microcapsule Artificial Kidney:  Including Updated Preparative Procedures and Properties" found in Kidney International, 10: S218-S214 (1976).  Preferably, the sorbent granules are encapsulated with a plastic such as collodion, nylon or other organic polymer.

Other techniques for the encapsulation of charcoal and other sorbents with nylon, collodion or other semipermeable materials may also be used as desired.  See particularly the book by Dr. Chang entitled Artificial Cells, published by Charles C. Thomas, Springfield, Illinois (1972).

Typically, 5 to 100 grams of sorbent may be present per liter of dialysis solution, although up to 300 grams may be used if desired of some grades of sorbent.

If desired, urease enzyme may be added to the system, for example, to convert urea into ammonia, an ammonia absorbant being provided.  Other sorbents may be used as well, being coated for example with a blood-compatible protein such as albumen or collagen in sufficiently thin layers so as not to seriously interfere with diffusion through the semipermeable polymer membrane.  Typically encapsulating membrane thicknesses may be in the range of 100 to 1,000 angstroms and effective pore sizes in the range of 5 to 45 angstroms may be utilized.

The following examples illustrate specific embodiments for testing the invention of this application.

### Example 1

A 0.6 $m^2$ cellulose acetate hollow fiber dialyzer, with its outer shell removed, was submerged in a transparent plastic container simulating a peritoneal cavity, containing two liters of dialyzate solution. The specific dialyzate used was Dianeal® peritoneal dialysis solution, sold by Travenol Laboratories, Inc. of Deerfield, Illinois and containing in some instances 1.5 percent by weight dextrose, and in other instances 4.25 percent by weight dextrose, as an osmotic agent for control of ultrafiltration. The dialyzate was warmed to $37^{\circ}$ C. in the container.

Perfusate, warmed to $37^{\circ}$ C., was pumped single pass by a roller pump through the hollow fiber dialyzer. The perfusate was a water solution of 140 MEq/liter sodium chloride and 12 mg. per dl. creatinine in deonized water.

Perfusate inflow and outflow of pressures was measured with positive pressure manometers, positioned at the liquid level of the dialyzate. Perfusate inflow rate and outflow rate were determined by timed collections into a graduated cylinder. The perfusate was pumped at 100 ml. per minute, and the inflow and outflow rates were measured at the beginning and end of each study. Assays of creatinine concentrations were performed with an Auto Analyzer® II sold by Technicon Instruments Corp.

To dialyzates used in this experimental system, various amounts of charcoal were added to determine its effect on creatinine clearance from the perfusate through the semipermeable membrane of the hollow fiber dialyzer into the dialyzate. Charcoal-free dialyzate was also tested for control purposes in the various experiments.

Large particles of activated charcoal measuring 850 to 2,000 microns in diameter (obtained from Fisher Scientific Company of Pittsburgh, Pennsylvania) were washed vigorously for three hours in cold running tap water to remove fine powder and soluble materials.

The large charcoal particles were then dried overnight in a ventilated oven at $350^{\circ}$ C.

The large charcoal particles were encapsulated in the manner similar to the method of Chang described in the article from _Kidney International_ cited above. First, a polymer solution was made by mixing 30 ml. of ethanol and 400 ml. of diethyl ether with 20 ml. of collodion. Next, 400 grams of the charcoal particles were added, and stirred quickly with a metal stirring rod to assure that all particles were coated. The polymer-coated charcoal particles were then spread out on aluminum foil, placed in a ventilated oven at $50^{\circ}$ C. and dried for five hours. The charcoal was then washed with deonized water, with care not to damage the polymer coatings. The charcoal particles were left in the deonized water in an airtight container, and refrigerated until use.

Alternatively, small charcoal particles of 1 to 2 microns diameter (Mallinckrodt Chemical Co., St. Louis, Missouri) were used without coating.

The respective charcoal particles were added to dialyzates to improve their function.

The dialyzers used were initially perfused with deonized water at $37^{\circ}$ C. against a water bath for two hours at pressures and flow rates similar to the rest of the studies to determine the integrity of the fibers and the amount of ultrafiltration due to hydrostatic pressure.

As a control, the three hour studies were performed with two liters of the dialyzates described above respectively containing 1.5 percent and 4.25 percent dextrose. Samples of perfusate outflow and dialyzate were assayed periodically, to determine free creatinine concentrations for calculation of creatinine clearance.

Through experiments performed in the manner described above, the following creatinine clearances from the perfusate into the dialyzate were obtained as shown in Table 1 below. Creatinine clearances were determined by analysis of small samples of dialysis solution passing through the membrane after 120 and 180 minutes of dialysis respectively. Large charcoal particles of 850 to 2,000 microns diameter were used in this experiment.

TABLE I

| Type of Dialyzate | Clearance of Creatinine After 120 Minutes of Dialysis (ml./min.) | Clearance of Creatinine After 180 Minutes of Dialysis (ml./min.) |
|---|---|---|
| Dialyzate with 1.5 percent dextrose and no charcoal | 2.9 | 2.9 |
| Dialyzate with 1.5 percent dextrose containing 70 grams of large particle charcoal without encapsulation | 7.6 | 9.3 |
| The same dialyzate with 70 grams of large particle charcoal encapsulated with collodion | 6.8 | 5.1 |
| The same dialyzate with 300 grams of large particle charcoal encapsulated in collodion | 7.4 | 6.4 |

8

9 0089135

## Example 2

The experiment of Example 1 was repeated, testing dialyzates which contained small particle size charcoal of 1 to 2 microns diameter. The data is as shown in Table II below:

TABLE II

| Type of Dialyzate | Clearance of Creatinine After 120 Minutes of Dialysis (ml./min.) | Clearance of Creatinine After 180 Minutes of Dialysis (ml./min.) |
|---|---|---|
| Dialyzate with 1.5 percent dextrose and no charcoal | 9.1 | 7.4 |
| Dialyzate with 1.5 percent dextrose plus 30 grams of small charcoal particles | 31.4 | 26.3 |
| Dialyzate containing 1.5 dextrose plus 20 grams of small charcoal particles | 26.1 | 21.4 |
| Dialyzate containing 4.25 percent dextrose and no charcoal | 10.3 | 10.3 |
| Dialyzate containing 4.25 percent dextrose plus 20 grams of small charcoal particles | 24.0 | 21.4 |

10

Example 3

Through experiments performed in the manner described in Example 1, the following data for mass transfer of creatinine from the perfusate into the dialyzate was obtained as shown in Table III below, using small charcoal particles (1 to 2 micron without encapsulation):

TABLE III

| Type of Dialyzate Used (2 liter) | Total Mass Transfer of Creatinine (mg.) after 3 Hour Exchange |
|---|---|
| Dialyzate with 1.5 percent dextrose - no charcoal | 29 |
| Dialyzate with 1.5 percent dextrose plus 30 grams of small charcoal particles | 713 |
| Dialyzate with 1.5 percent dextrose plus 20 grams of small charcoal particles | 723 |
| Dialyzate with 4.25 percent dextrose - no charcoal | 393 |
| Dialyzate with 4.25 percent dextrose plus 20 grams of small charcoal particles | 628 |

The large charcoal particles showed no significant improvement in total mass transfer in this experiment, in part because of their lower surface area available for absorption, but apparently also as an artifact of the particular experiment.

## Example 4

Ultrafiltration measurements were also made on various of the solutions tested as in Example 1, with the results being as shown in Table IV, using large charcoal particles (850 to 2,000 microns):

### TABLE IV

| Dialyzate Used (2 liters) | Total Ultrafiltration over 3 Hour Run (ml.) |
|---|---|
| Dialyzate solution containing 1.5 percent dextrose | 76 |
| Dialyzate containing 1.5 percent dextrose plus 70 grams of large charcoal particles | 96 |
| Dialyzate containing 1.5 percent dextrose and 70 grams of large charcoal particles coated with collodion | 116 |
| Dialyzate containing 1.5 percent dextrose plus 300 grams of large charcoal particles coated with collodion | 148 |

Thus it can be seen that there is a significant increase in the clearance of creatinine and related materials in a dialysis situation when charcoal particles are included in the dialyzate, with the charcoal particles being preferably coated with collodion or the like.

It is also noted that the presence of the charcoal, particularly when coated with collodion or other plastic membrane, tends to increase the ultrafiltration. This may be a valuable feature in those instances where it is desired to remove more water from the patient into the peritoneal dialysis solution, while providing low concentrations of sugar to the patient.

Thus the solution of this invention may be passed into the peritoneal cavity by means of a Tenckoff catheter or the like. Preferably, the peritoneal dialysis procedure may be overall a conventional procedure in accordance with the teachings of U.S. Patent No. 4,239,041, for example, or in accordance with another conventional procedure for effecting peritoneal dialysis, but with the presence of the preferably encapsulated absorbent granules for the purpose of increasing the overall mass transfer of particularly lower molecular weight materials from the bloodstream to the dialysis solution, for improved well being of the patient.

The above has been offered for illustrative purposes only, and is not intended to limit the scope of the invention of this application, which is as defined in the claims below.

14

CLAIMS

1. A peritoneal dialysis solution which contains a sufficient amount of physiological salt to be osmotically compatible, characterised in that it also contains from 5 to 100 grams per liter of a particulate sorbent for metabolic breakdown products to be removed having a particle size of 0.5 to 2,000 microns.

2. A peritoneal dialysis solution according to Claim 1 containing from 5 to 50 grams per liter of the particulate sorbent.

3. A peritoneal dialysis solution according to Claim 1 or 2 in which the particle size of said sorbent is 1 to 100 microns.

4. A peritoneal dialysis solution according to Claim 1 or 2 in which the particle size of said sorbent is 1 to 20 microns.

5. A peritoneal dialysis solution according to any preceding claim in which said sorbent is carbon and preferably charcoal.

6. A peritoneal dialysis solution according to any preceding claim in which the sorbent is encapsulated with a semipermeable polymeric layer.

7. The method of performing peritoneal dialysis which comprises inserting into the peritoneal cavity a peritoneal dialysis solution which includes a solid particulate sorbent for metabolic breakdown products to be removed; allowing said peritoneal dialysis solution to dwell for a period of time in the peritoneal cavity; and thereafter removing the peritoneal dialysis solution and particulate sorbent from the peritoneal cavity.

European Patent
Office

**EUROPEAN SEARCH REPORT**

EP 83 30 1046

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-3 525 686 (M. ROBERTS) * Claims 1, 7, 8; column 1, lines 21-35; column 2, lines 33-40 * | 1-4 | A 61 M 1/03 |
| Y | US-A-3 463 728 (T. KOLOBOW et al.) * Claims 1, 8-11 * | 1-4 | |
| D,Y | T.M.S. CHANG "Artificial cells", 1972, CHARLES C. THOMAS, Springfield pages 70-77, 114-115 * Whole document, in particular page 114, line 16 * | 1-4 | |
| D,A | US-A-3 725 113 (T.M.S. CHANG) * Claims 1, 2 * | 5,6 | |
| D,A | US-A-4 239 041 (R.P. POPOVICH et al.) * Claim 5 * | 7 | TECHNICAL FIELDS SEARCHED (Int. Cl. ³) A 61 M 1/03 B 01 D 13/00 B 01 D 15/00 |
| A | DE-A-2 741 888 (AKZO GMBH) * Claim 3; page 3, last paragraph; page 4, 1st and 2nd paragraphs * | 1 | |
| P,A | EP-A-0 064 393 (PURDUE RESEARCH FOUNDATION) * Claims 1, 2, 10, 11; page 3, lines 21-23 * | 1,5,7 | |

The present search report has been drawn up for all claims

| Place of search BERLIN | Date of completion of the search 08-06-1983 | Examiner CLOT P.F.J. |
|---|---|---|